(19) Europäisches Patentamt
European Patent Office
Office européen des brevets

(11) Veröffentlichungsnummer: **0 258 782**
**A2**

(12) # EUROPÄISCHE PATENTANMELDUNG

(21) Anmeldenummer: 87112283.4

(51) Int. Cl.4: **A61K 31/41**

(22) Anmeldetag: 25.08.87

(30) Priorität: 04.09.86 DE 3630130

(43) Veröffentlichungstag der Anmeldung:
09.03.88 Patentblatt 88/10

(84) Benannte Vertragsstaaten:
AT BE CH DE ES FR GB IT LI NL SE

(71) Anmelder: **BAYER AG**
**Konzernverwaltung RP Patentabteilung**
**D-5090 Leverkusen 1 Bayerwerk(DE)**

(72) Erfinder: **Büchel, Karl Heinz, Prof. Dr.**
**Dabringhausener Strasse 42**
**D-5093 Burscheid(DE)**
Erfinder: **Holmwood, Graham, Dr.**
**Krutscheider Weg 105**
**D-5600 Wuppertal 11(DE)**
Erfinder: **Kraatz, Udo, Dr.**
**Andreastrasse 22a**
**D-5090 Leverkusen 1(DE)**
Erfinder: **Krämer, Wolfgang, Dr.**
**Rosenkranz 25**
**D-5093 Burscheid 2(DE)**
Erfinder: **Plempel, Manfred, Dr.**
**Zwengenbergstrasse 3c**
**D-5657 Haan(DE)**

(54) **Verwendung von Mercapto-substituierten Hydroxyethyl- (triazol-1-yl)-Derivaten zur Behandlung von Krankheiten.**

(57) Die Erfindung betrifft mercapto-substituierte Hydroxyethyl-(triazol-1-yl)-Derivate der allgemeinen Formel (I)

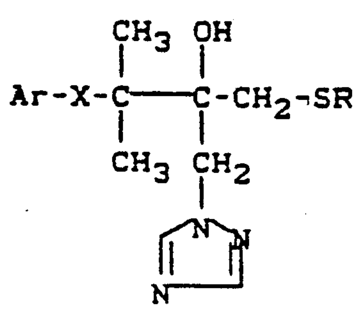

(I)

mit den in der Beschreibung angegebenen Substituentendefinitionen,
ein Verfahren zu ihrer Herstellung und ihre Verwendung als antimikrobielle Mittel, insbesondere als Antimykotika.

EP 0 258 782 A2

# Verwendung von Mercapto-substituierten Hydroxyethyl -(triazol-1-yl)-Derivaten zur Behandlung von Krankheiten

Die vorliegende Erfindung betrifft die Verwendung von neuen mercapto-substituierten Hydroxyethyl-(triazol-1-yl)-Derivaten zur Behandlung von Krankheiten; ihre Verwendung als antimikrobielle Mittel, insbesondere Antimykotika sowie ein Verfahren zu ihrer Herstellung.

Es ist bereits bekannt geworden, daß bestimmte 1-Hydroxyalkyl-triazolyl-Derivate, wie beispielsweise 3-(4-Chlorphenyl)-2-(2,4-dichlorphenoxy)-3,3-dimethyl-1-(1,2,4-triazol-1-yl)-propan-2-ol und 3-(2,4-Dichlorphenoxy)-2-(4-chlorphenyl-methyl)-3,3-dimethyl-1-(1,2,4-triazol-1-yl)-propan-2-ol gute antimykotische Eigenschaften aufweisen (vergleiche DE-OS 32 02 613). Die Wirkung dieser Verbindungen ist jedoch nicht immer voll befriedigend.

Es wurde gefunden, daß die neuen mercapto-substituierten Hydroxyethyl-(triazol-1-yl)-Derivate der Formel

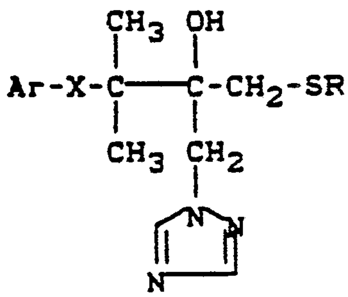

(I)

in welcher

Ar für gegebenenfalls substituiertes Aryl steht,

X für Sauerstoff, Schwefel, eine direkte Bindung oder für die Gruppierungen $-CH_2-$, $-CH_2-CH_2-$, $-O-CH_2-$, $-SCH_2-$, $-O-CH_2-CH_2-$oder $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2-$steht, und

R für Wasserstoff, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl oder für gegebenenfalls substituiertes Aralkyl oder gegebenenfalls substituiertes Phenyl steht.

sowie deren Säureadditions-Salze und Metallsalz-Komplexe gute antimikrobielle, insbesondere antimykotische Eigenschaften aufweisen.

Die neuen mercapto-substituierten Hydroxyethyl-(triazol-1-yl)-Derivate der Formel (I) besitzen ein asymmetrisch substituiertes Kohlenstoffatom und können deshalb in den beiden optischen Isomerenformen anfallen. Die Erfindung betrifft sowohl die Isomerengemische als auch die einzelnen Isomeren.
Überraschenderweise besitzen die erfindungsgemäß zu verwendenden mercapto-substituierten Hydroxyethyl-(triazol-1-yl)-Derivate der Formel (I) sowie deren Säureadditions-Salze und Metallsalz-Komplexe in bestimmten Indikationsbereichen ein besseres Wirkungsspektrum, als die aus dem Stand der Technik bekannten 1-Hydroxyalkyl-triazol-Derivate wie beispielsweise 3-(4-Chlorphenyl)-2-(2,4-dichlorphenoxy-3,3-dimethyl-1-(1,2,4-triazol-1-yl)-propan-2-ol und 3-(2,4-Dichlorphenoxy)-2-(4-chlorphenyl-methyl)-3,3-dimethyl-1-(1,2,4-triazol-1-yl)-propan-2-ol, welche chemisch und wirkungsmäßig naheliegende Verbindungen sind. Die erfindungsgemäße Verwendung der Stoffe der Formel (I) stellt somit eine Bereicherung der Pharmazie dar.

Die erfindungsgemäß zu verwendenden mercapto-substituierten Hydroxyethyl-(triazol-1-yl)-Derivate sind durch die Formel (I) allgemein definiert. Bevorzugt sind diejenigen Verbindungen der Formel (I), in denen

Ar für Aryl mit 6 bis 10 Kohlenstoffatomen steht, welches einfach oder mehrfach, gleichartig oder verschieden substituiert sein kann durch Halogen, gegebenenfalls substituiertes Alkyl, gegebenen falls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone,

X für Sauerstoff, Schwefel, eine direkte Bindung oder für die Gruppierungen $-CH_2-$, $-CH_2-CH_2-$, $-O-CH_2-$, $-SCH_2-$, $-O-CH_2-CH_2-$oder $-S-CH_2-CH_2-$steht, wobei das Heteroatom mit dem Aryl-Rest verbunden ist, wenn X für $-OCH_2-$, $-SCH_2-$, $-O-CH_2-$oder $-S-CH_2-CH_2-$steht, und

R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlenstoffatomen, gegebenenfalls substituiertes Alkinyl mit 3 bis 8 Kohlenstoffatomen, gegebenenfalls einfach oder zweifach, gleichartig oder verschieden durch Halogen und/oder Alyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil ofer für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone, oder für ggfls. durch Halogen und/oder Alkyl substituiertes Phenyl steht.

Eine besonders bevorzugte Gruppe der erfindungsgemäß zu verwendenden Stoffe sind diejenigen mercapto-substituierten Hydroxyethyl-(triazol-1-yl)-Derivate der Formel (I), in denen

Ar für Phenyl steht, welches einfach bis dreifach, gleichartig oder verschieden substituiert sein kann durch Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen und/oder Phenyl.

X für Sauerstoff, Schwefel, eine direkte Bindung oder für die Gruppierungen $-CH_2$-oder $-CH_2-CH_2$-steht, und

R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlenstoffatomen, gegebenenfalls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone, oder R für ein-bis dreifach durch Halogen und/oder Alkyl ($C_1$-$C_4$) substituiertes Phenyl steht.

Eine weitere besonders bevorzugte Gruppe der erfindungsgemäß zu verwendenden Stoffe sind diejenigen mercaptosubstituierten Hydroxyethyl-(triazol-1-yl)-Derivate der Formel (I), in denen

Ar für Phenyl steht, welches einfach bis dreifach, gleichartig oder verschieden substituiert sein kann durch Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl, Formyl und Acetyl und/oder von Formyl oder Acetyl abgeleitete Oximether,

X für die Gruppierungen $-O-CH_2$-, $-S-CH_2$-, $-O-CH_2-CH_2$-oder $-S-CH_2-CH_2$-steht, wobei das Heteroatom jeweils mit dem Aryl-Rest verbunden ist, und

R für Wasserstoff, gegebenenfalls substituiertes, geradkettiges oder verzweigtes Alkyl mit 1 bis 12 Koylenstoffatomen, gegebenenfalls substituiertes Alkenyl mit 2 bis 12 Kohlenstoffatomen, gegebenenfalls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone, oder R für ein-bis dreifach durch Halogen und/oder Alkyl ($C_1$-$C_4$) substituiertes Phenyl steht.

Ganz besonders bevorzugt ist die Verwendung solcher Verbindungen der Formel (I), in denen

Ar für Phenyl steht, welches einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto und/oder Phenyl,

X für Sauerstoff oder für die $-CH_2$-Gruppe steht und

R für Wasserstoff, gegebenenfalls durch Halogen substituiertes Alkyl mit 1 bis 6 Kohlenstoffatomen, gegebenenfalls durch Halogen substituiertes Alkenyl mit 2 bis 4 Kohlenstoffatomen, gegebenenfalls durch Halogen substituiertes Alkinyl mit 3 bis 6 Kohlenstoffatomen, gegebenenfalls ein-oder zweifach, gleichartig oder verschieden durch Fluor, Chlor und/oder Methyl substituiertes Cycloalkyl mit 3 bis 6 Kohlenstoffatomen, gegebenenfalls ein-oder zweifach, gleichartig oder verschieden durch Fluor, Chlor und/oder Methyl substituiertes Cycloalkylalkyl mit 3 bis 6 Kohlenstoffatomen im Cycloalkylteil und 1 oder 2 Kohlenstoffatomen im Alkylteil oder für Benzyl, welches im Phenylteil einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto und/oder Tetrafluorethylmercapto oder R für ggfls. ein-oder zweifach durch Fluor, Chlor und/oder Methyl substituiertes Phenyl steht.

Ganz besonders bevorzugt sind Verbindungen der Formel (I) in denen

Ar für Phenyl steht, welches einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, Formyl, Acetyl, Methoximinomethyl, Ethoximinomethyl, Methoximinoethyl, Ethoximinoethyl und/oder Phenyl,

X für die Gruppierungen $-O-CH_2-$, $-S-CH_2-$, $-O-CH_2-CH_2-$oder $-S-CH_2-CH_2-$steht, wobei das Heteroatom mit dem Arylrest verbunden ist, und

R für Wasserstoff, gegebenenfalls durch Halogen substituiertes Alkyl mit 1 bis 6 Kohlenstoffatomen, gegebenenfalls durch Halogen substituiertes Alkenyl mit 2 bis 4 Kohlenstoffatomen, gegebenenfalls durch Halogen substituiertes Alkinyl mit 3 bis 6 Kohlenstoffatomen, gegenbenenfalls ein-oder zweifach, gleichartig oder verschieden durch Fluor, Chlor und/oder Methyl substituiertes Cycloalkyl mit 3 bis 6 Kohlenstoffatomen, gegebenenfalls ein-oder zweifach, gleichartig oder verschieden durch Fluor, Chlor und/oder Methyl substituiertes Cycloalkylalkyl mit 3 bis 6 Kohlenstoffatomen im Cycloalkylteil und 1 oder 2 Kohlenstoffatomen im Alkylteil oder für Benzyl, welches im Phenylteil einfach bis dreifach, gleichartig oder verschieden substituiert sein kann durch Fluor, Chlor, Brom, Methyl, Ethyl, Isopropyl, Butyl, Trifluormethyl, Trifluoromethoxy, Tetrafluorethoxy, Trifluormethylmercapto und/oder Tetrafluorethylmercapto oder R für ggfls. ein-oder zweifach durch Fluor, Chlor und/oder Methyl substituiertes Phenyl steht.

Von ganz besonderem Interesse sind weiterhin solche Verbindungen der Formel (I), in denen

Ar für Phenyl steht, welches einfach oder zweifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, Formyl, Acetyl, Methoximinomethyl, Ethoximinomethyl, Methoximinoethyl, Ethoximinoethyl und/oder Phenyl,

X für die Gruppierungen $-O-CH_2-$oder $-O-CH_2-CH_2-$steht, wobei das Heteroatom mit dem Arylrest verbunden ist, und

R für Wasserstoff, Methyl, Ethyl, i-Propyl, n-Propyl, i-Butyl, n-Butyl, t-Butyl, n-Hexyl, Cyclohexyl, Allyl, Propinyl, Dichlorcyclopropyl, Dichlorcyclopropyl-methyl, Methylcyclohexyl, 4-Chlorbenzyl oder 4-Chlorphenyl steht.

Von besonderem Interesse sind Verbindungen der Formel (I) in denen

Ar für Phenyl steht, welches einfach oder zweifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, und/oder Phenyl,

X für Sauerstoff oder für die Gruppierung $-CH_2$ steht und

R für Wasserstoff, Methyl, Ethyl, i-Propyl, n-Propyl, i-Butyl, n-Butyl, t-Butyl, n-Hexyl, Cyclohexyl, Allyl, Propinyl, Dichlorcyclopropyl, Dichlorcyclopropylmethyl, Methylcyclohexyl, M-Chlorbenzyl oder 4-Chlorphenyl steht.

Bevorzugte erfindungsgemäß zu verwendende Verbindungen sind auch Additionsprodukte aus Säuren und denjenigen mercapto-substituierten Hydroxyethyl-(triazol-1-yl)-Derivaten der Formel (I), in denen Ar, X und R diejenigen Bedeutungen haben, die im Zusammenhang mit .der Beschreibund der erfindungsgemäßen Stoffe bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäß zu verwendende Verbindungen sind Additionsprodukte aus Salzen von Metallen der II. bis IV. Haupt-und der I. und II. sowie IV. bis VIII. Nebengruppe des Periodensystems der Elemente und denjenigen mercapto-substituierten Hydroxyethyl-(triazol-1-yl)-Derivaten der Formel (I), in denen Ar, X und R die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhand die Halogenwasserstoffsäuren, wie z.B. die Chlorwasserstoffsäure und die Bromwasserstoffsäure, ferner Phosphorsäure, Salpetersäure und Schwefelsäure.

Die Derivate der Formel (I) können erhalten werden, indem man Oxirane der Formel (II)

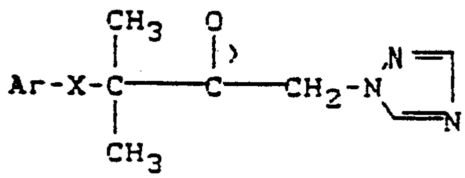

(II)

in welcher
Ar und X die oben angegebene Bedeutung haben,
mit Mercaptanen der Formel
R-SH (III)
in welcher
R die oben angegebene Bedeutung hat,
in Gegenwart eines Verdünnungsmittels, wie beispielsweise Alkohole, gegebenenfalls in Gegenwart eines Säurebindemittels wie beispielsweise Alkalihydroxid bei Temperaturen zwischen 0 °C und 200 °C umsetzt und gegebenenfalls anschließend an die so erhaltenen Verbindungen der Formel (I) eine Säure oder ein Metallsalz addiert.

Die Oxirane der Formel (II) sind neu. Sie lassen sich herstellen, indem man Ketone der Formel

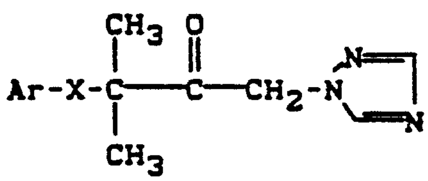

(IV)

in welcher
Ar und X die oben angegebene Bedeutung haben,
entweder
α) mit Dimethyloxosulfonium-methylid der Formel

$$\overset{\delta^+\delta^-}{(CH_3)_2SOCH_2} \qquad (V)$$

in Gegenwart eines Verdünnungsmittels, wie beispielsweise Dimethylsulfoxid, bei Temperaturen zwischen 20°C und 80°C umsetzt,
oder
β) mit Trimethylsulfonium-methylsulfat der Formel

$[(CH_3)_3S^{\oplus}]\ CH_3SO_4^{\ominus}$ (VI) in Gegenwart eines inerten organischen Lösungsmittels, wie z.B. Acetonitril, und in Gegenwart einer Base, wie z.B. Natriummethylat, bei Temperaturen zwischen 0°C bis 60°C, vorzugsweise bei Raumtemperatur, umsetzt.

Die Oxirane der Formel (II) können gegebenenfalls ohne Isolierung direkt weiter umgesetzt werden.

Die bei der Herstellung der Oxirane der Formel (II) nach den obigen Verfahren als Ausgangsstoffe benötigten Ketone der Formel (IV) sind bekannt oder lassen sich nach prinzipiell bekannten Methoden in einfacher Weise herstellen (vgl. EP-OS 0 054 865 und DE-OS 3 222 220).

Die außerdem als Ausgangsstoffe benötigten Mercaptane sind durch die Formel (III) allgemein definiert. In dieser Formel steht R vorzugsweise für die Bedeutungen, die bereits im Zusammenhang mit der Beschreibung der erfindungsgemäß zu verwendenden Stoffe der Formel (I) bevorzugt für R genannt wurden.

Die Mercaptane der Formel (III) sind allgemein bekannte Verbindungen der organischen Chemie.

Die erfindungsgemäß zu verwendenden Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäß zu verwendenden Säureadditions-Salze als bevorzugte Säuren genannt wurden.

Die Säureadditions-Salze der Verbindingen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, Isolieren und gegebenenfalls durch Umkristallisation reinigen.

Die erfindungegemäß zu verwendenden Verbindungen der Formel (I) und ihre Säureadditions-Salze weisen antimikrobielle, insbesondere starke antimykotische Wirkungen auf. Sie besitzen ein sehr breites antimykotisches Wirkungssprektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten sowie Epidermophotyn floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll-und Streckmittel, z.B. Stärken, Milchzucker, Rohrzucker, Glucose, Mannit und Kieselsäure re, (b) Bindemittel, z.B. Carboxymethylcellulose, Alginate, Gelantine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium-und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Träger stoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silocone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit-und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human-und Veterinärmedizin aur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human-als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

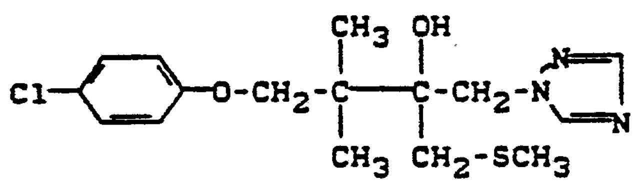

(I-1)

Zu 18 g (0,042 Mol) 2-(4-Chlor-phenoxy-tert.-butyl)-2-(1,2,4-triazol-1-yl-methyl)-oxiran in 80 ml Ethanol werden unter Rühren 1,8 g Natriumhydroxid in 80 ml Ethanol gegeben. Der Ansatz wird gekühlt und bei -15°C werden 2,4 g (0,055 Mol) Methylmercaptan eingeleitet. Anschließend läßt man das Reaktionsgemisch langsam auf Raumtemperatur kommen und rührt weitere 16 Stunden. Der Ansatz wird eingeengt, das verbleibende Öl in 350 ml Dichlormethan aufgenommen, mit zweimal 500 ml Wasser gewaschen, über Natriumsulfat getrocknet und eingeengt. Der Rückstand wird über eine Kieselgelsäule (Laufmittel: Cyclohexan/Essigester 1:1) gereinigt.

Man erhält 12g (80 % der Theorie) 2-(Methylthiomethyl)-3,3-dimethyl-4-(4-chlor-phenoxy)-1-(1,2,4-triazol-1-yl)-butan-2-ol als zähes Öl. Die Struktur der Verbindung wird durch das Kernresonanzspektrum belegt.

### Herstellung von Ausgangsprodukten

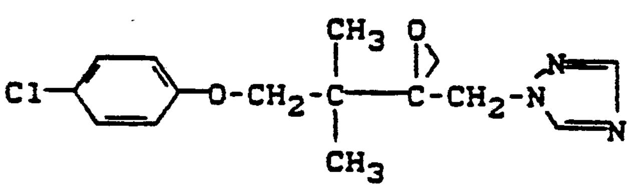

(II-1)

In eine Lösung von 170 ml absolutem Dimethylsulfoxid und 78,4 g Kalium-t-Butylat werden 154 g (0,7 Mol) Trimethylsulfoxoniumjodid gegeben. Der Ansatz wird 5 Stunden gerührt, danach tropfenweise mit 182 g (0,7 Mol) 1-(1,2,4-Triazol-1-yl)-4-(4-chlor-phenoxy)-3,3-dimethylbutan-2-on versetzt und 16 Stunden bei 45°C gerührt. Nach der Zugabe von 300 ml Wasser wird die organische Phase abgetrennt und die Wasser-Phase einmal mit 500 ml Dichlormethan extrahiert. Die organischen Phasen werden vereinigt und zweimal mit 1200 ml Wasser gewaschen. Nach dem Trocknen über Natriumsulfat wird das Verdünnungsmittel abgezogen und der Rückstand im Hockvakuum entgast.

Man erhält 187 g (86,8 % der Theorie) 2-(4-Chlor-phenoxy-tert.-butyl)-2-(1,2,4-triazol-1-yl-methyl)-oxiran als Öl.

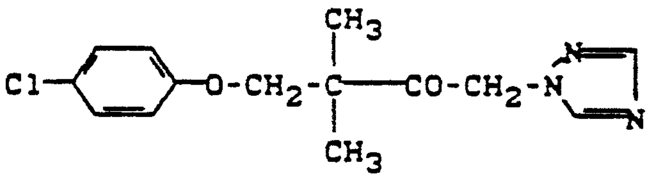

(IV-1)

Zu einem Gemisch aus 138 g (1 Mol) Kaliumcarbonat und 50,4 g (0,73 Mol) 1,2,4-Triazol in 800 ml absolutem siedenden Aceton werden 222 g (0,73 Mol) 1-Brom-4-(4-chlor-phenoxy)-3,3-dimethyl-butan-2-on, gelöst in Aceton, unter Rühren zugetropft.

Man erwärmt 5 Stunden unter Rückfluß, kühlt auf 0 °C bis 10°C ab, filtriert vom Salz ab und engt das Filtrat unter vermindertem Druck ein. Der Rückstand wird in 800 ml Dichlormethan aufgenommen und zweimal mit 1300 ml Wasser gewaschen. Die organische Phase wird über Natriumsulfat getrocknet und eingeengt.

Man erhält 182 g (85 % der Theorie) 1-(1,2,4-Triazol-1-yl)-4-(4-chlor-phenoxy)-3,3-dimethylbutan-2-on in Form farbloser Kristalle vom Schmelzpunkt 88-90°C.

In analoger Weise zu der im Beispiel 1 beschriebenen Methode und unter Berücksichtigung der Angaben zu dem erfindungsgemäßen Verfahren werden die nachfolgend aufgeführten Verbindung der Formel

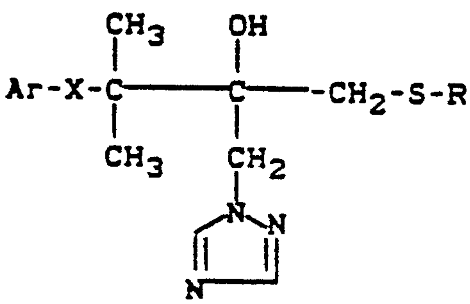

(I)

erhalten:

<u>Tabelle 1</u>

| Bsp. Nr. | Verb. Nr. | Ar | X | R | Schmelzpunkt [°C] bzw. Brechungsindex [$n_D^{20}$] |
|---|---|---|---|---|---|
| 2 | I-2 | 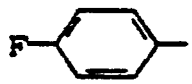 | $-CH_2$ | $C_2H_5$ | Öl |
| 3 | I-3 | 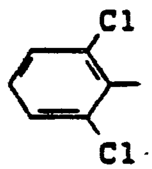 | $-O-CH_2$ | $nC_3H_7$ | Öl |
| 4 | I-4 | 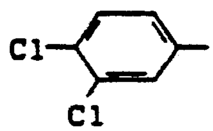 | $-O-CH_2$ | $C_2H_5$ | 85 |
| 5 | I-5 | 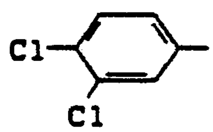 | $-O-CH_2$ | $iC_3H_7$ | 94-97 |
| 6 | I-6 | 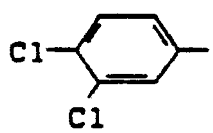 | $-O-CH_2$ | 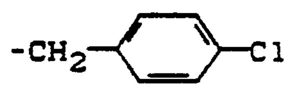 | 92-94 |
| 7 | I-7 | 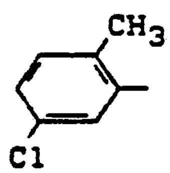 | $-O-CH_2$ | $C_2H_5$ | Öl |
| 8 | I-8 | 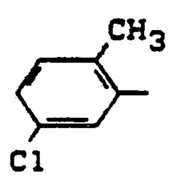 | $-O-CH_2$ | $nC_4H_9$ | Öl |
| 9 | I-9 | 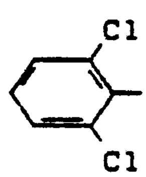 | $-O-CH_2$ | $nC_4H_9$ | Öl |

<u>Tabelle 1</u> (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 10 | I-10 | 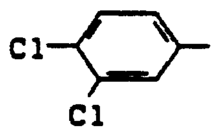 | $-O-CH_2$ | $nC_3H_7$ | 71-73 |
| 11 | I-11 | 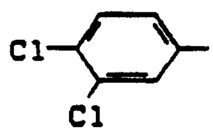 | $-O-CH_2$ | $nC_4H_9$ | Öl |
| 12 | I-12 | 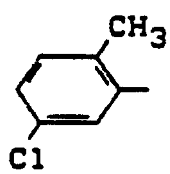 | $-O-CH_2$ | $nC_3H_7$ | Öl |
| 13 | I-13 | 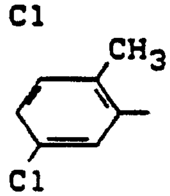 | $O-CH_2$ | $iC_3H_7$ | 86-99 |
| 14 | I-14 | 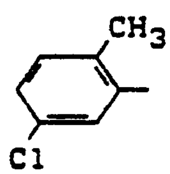 | $O-CH_2$ | 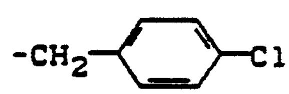 | Öl |
| 15 | I-15 | 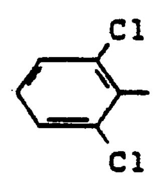 | $O-CH_2$ | $C_2H_5$ | Öl |
| 16 | I-16 | 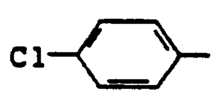 | $O-CH_2$ | 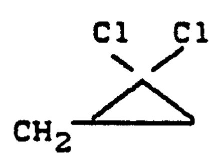 | Öl |

Tabelle 1 (Fortsetzung)

| Bsp. Nr. | Verb. Nr. | Ar | X | R | Schmelzpunkt [°C] bzw. Brechungsindex $[n_D^{20}]$ |
|---|---|---|---|---|---|
| 17 | I-17 | 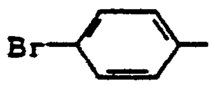 | O-$CH_2$ | 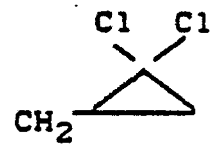 | Öl |
| 18 | I-18 | 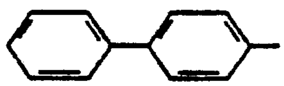 | O-$CH_2$ | 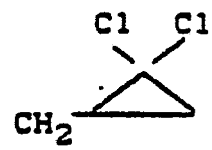 | Öl |
| 19 | I-19 | 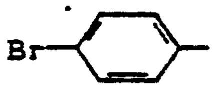 | O-$CH_2$ | $C_2H_5$ | Öl |
| 20 | I-20 | 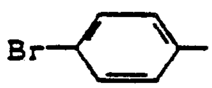 | O-$CH_2$ | $nC_3H_7$ | Öl |
| 21 | I-21 | 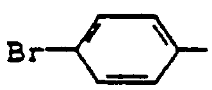 | O-$CH_2$ | $iC_3H_7$ | Öl |
| 22 | I-22 | 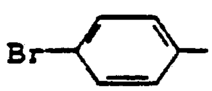 | O-$CH_2$ | $nC_4H_9$ | Öl |
| 23 | I-23 | 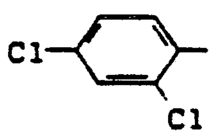 | O-$CH_2$ | $C_2H_5$ | Öl |
| 24 | I-24 | 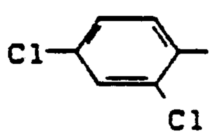 | O-$CH_2$ | $nC_3H_7$ | Öl |
| 25 | I-25 | 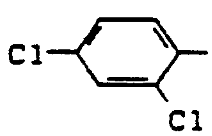 | O-$CH_2$ | $nC_4H_9$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 26 | I-26 | 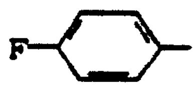 | $CH_2$ | $CH_2$ 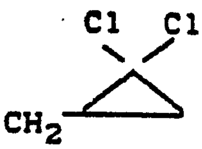 | Öl |
| 27 | I-27 | 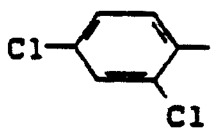 | $O-CH_2$ | $iC_3H_7$ | Öl |
| 28 | I-28 | 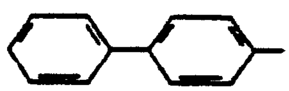 | $O-CH_2$ | $C_2H_5$ | Öl |
| 29 | I-29 | 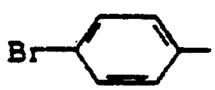 | $O-CH_2$ | $CH_3$ | 69-71 |
| 30 | I-30 | 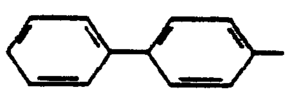 | $O-CH_2$ | $CH_3$ | 120-123 |
| 31 | I-31 | 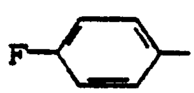 | $CH_2$ | $CH_3$ | 135 |
| 32 | I-32 | 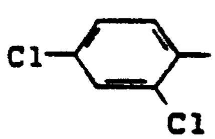 | $O-CH_2$ | $CH_3$ | Öl |
| 33 | I-33 | 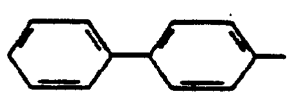 | $O-CH_2$ | $nC_4H_9$ | Öl |
| 34 | I-34 | 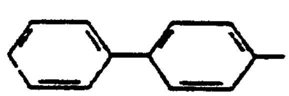 | $O-CH_2$ | $nC_3H_7$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 35 | I-35 | 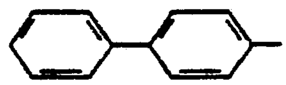 | O-$CH_2$ | i$C_3H_7$ | Öl |
| 36 | I-36 | 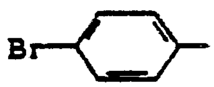 | $CH_2$ | -$CH_2$-CH=$CH_2$ | 108 |
| 37 | I-37 | 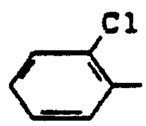 | $CH_2$ | i$C_3H_7$ | Öl |
| 38 | I-38 | 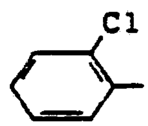 | $CH_2$ | -$CH_2$-CH=$CH_2$ | Öl |
| 39 | I-39 | 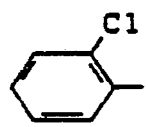 | $CH_2$ | $C_2H_5$ | Öl |
| 40 | I-40 | 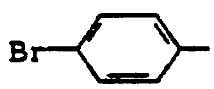 | $CH_2$ | i$C_3H_7$ | 80-81 |
| 41 | I-41 | 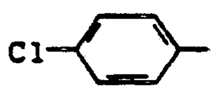 | $CH_2$ | n$C_3H_7$ | Öl |
| 42 | I-42 | 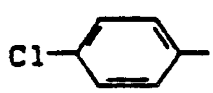 | $CH_2$ | $CH_3$ | 146 |
| 43 | I-43 | 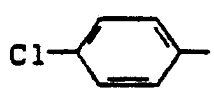 | $CH_2$ | i$C_4H_9$ | Öl |
| 44 | I-44 | 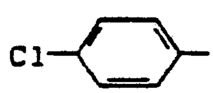 | $CH_2$ | -$CH_2$-CH=$CH_2$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 45 | I-45 | 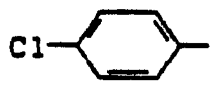 | $CH_2$ | $C_2H_5$ | 82-83 |
| 46 | I-46 | 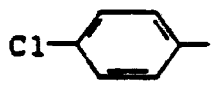 | $CH_2$ | $iC_3H_7$ | Harz |
| 47 | I-47 | 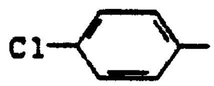 | $CH_2$ | $nC_4H_9$ | Harz |
| 48 | I-48 | 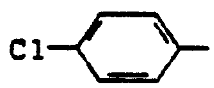 | $CH_2$ | $nC_6H_{13}$ | Harz |
| 49 | I-49 | 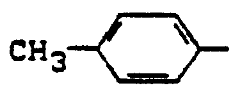 | $CH_2$ | $nC_3H_7$ | Öl |
| 50 | I-50 | 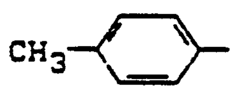 | $CH_2$ | $C_2H_5$ | 102-103 |
| 51 | I-51 | 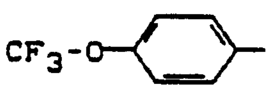 | $CH_2$ | $nC_3H_7$ | Öl |
| 52 | I-52 | 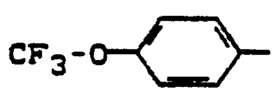 | $CH_2$ | $C_2H_5$ | Öl |
| 53 | I-53 | 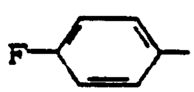 | $CH_2$ | $nC_3H_7$ | Öl |
| 54 | I-54 | 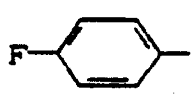 | $CH_2$ | $nC_4H_9$ | Öl |
| 55 | I-55 | 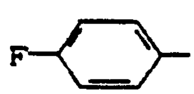 | $CH_2$ | $iC_3H_7$ | 76 |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 56 | I-56 | 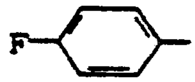 | $CH_2$ | H | Öl |
| 57 | I-57 | 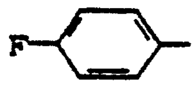 | $CH_2$ | $nC_6H_{13}$ | Öl |
| 58 | I-58 | 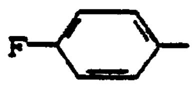 | $CH_2$ | 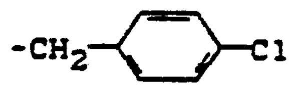 | Öl |
| 59 | I-59 | 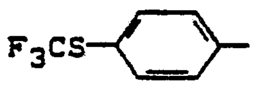 | $CH_2$ | $C_2H_5$ | Öl |
| 60 | I-60 | 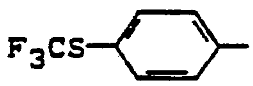 | $CH_2$ | $nC_3H_7$ | Öl |
| 61 | I-61 | 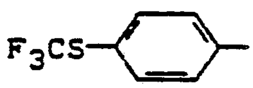 | $CH_2$ | $nC_4H_9$ | Öl |
| 62 | I-62 | 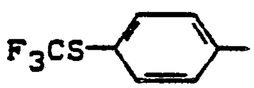 | $CH_2$ | $iC_3H_7$ | Öl |
| 63 | I-63 | 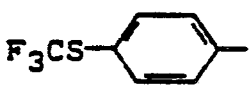 | $CH_2$ | H | Öl |
| 64 | I-64 | 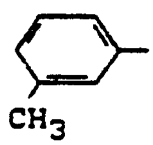 | $CH_2$ | $C_2H_5$ | 78 |
| 65 | I-65 | 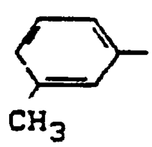 | $CH_2$ | $iC_3H_7$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 66 | I-66 | 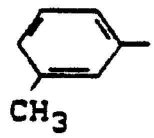 | $CH_2$ | $nC_4H_9$ | Öl |
| 67 | I-67 | 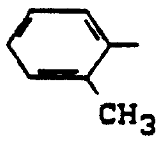 | $CH_2$ | $C_2H_5$ | Öl |
| 68 | I-68 | 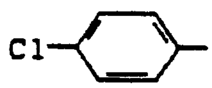 | $OCH_2$ | $C_2H_5$ | Öl |
| 69 | I-69 | 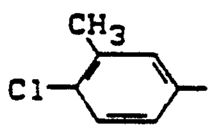 | $OCH_2$ | $C_2H_5$ | Öl |
| 70 | I-70 | 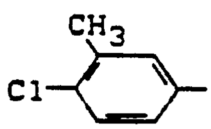 | $OCH_2$ | $nC_3H_7$ | Öl |
| 71 | I-71 | 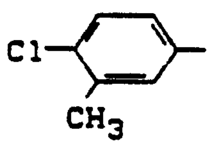 | $OCH_2$ | $nC_4H_9$ | Öl |
| 72 | I-72 | 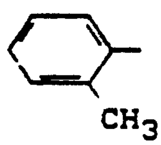 | $CH_2$ | $nC_3H_7$ | Öl |
| 73 | I-73 | 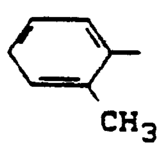 | $CH_2$ | $iC_3H_7$ | Öl |

Tabelle 1 (Fortsetzung)

| Bsp. Nr. | Verb. Nr. | Ar | X | R | Schmelzpunkt [°C] bzw. Brechungsindex $[n_D^{20}]$ |
|---|---|---|---|---|---|
| 74 | I-74 | 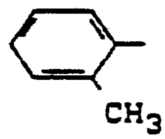 | $CH_2$ | $nC_4H_9$ | Öl |
| 75 | I-75 | 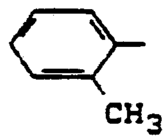 | $CH_2$ | 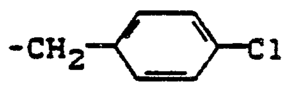 | Öl |
| 76 | I-76 | 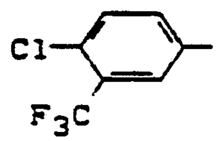 | $CH_2$ | $nC_4H_9$ | Öl |
| 77 | I-77 | 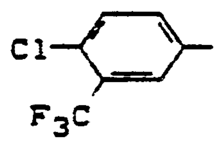 | $CH_2$ | $C_2H_5$ | Öl |
| 78 | I-78 | 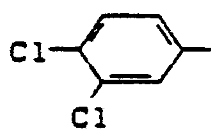 | $CH_2$ | $C_2H_5$ | Öl |
| 79 | I-79 | 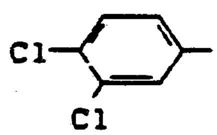 | $CH_2$ | $nC_3H_7$ | Öl |
| 80 | I-80 | 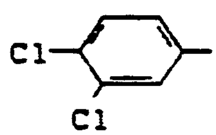 | $CH_2$ | $iC_3H_7$ | Öl |
| 81 | I-81 | 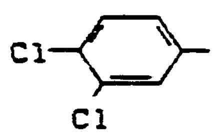 | $CH_2$ | $nC_4H_9$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 82 | I-82 | 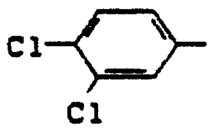 | $CH_2$ | 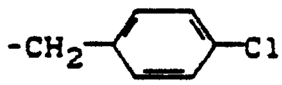 | Öl |
| 83 | I-83 | 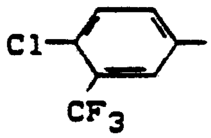 | $CH_2$ | $iC_3H_7$ | Öl |
| 84 | I-84 | 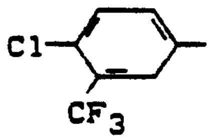 | $CH_2$ | $nC_3H_7$ | Öl |
| 85 | I-85 | 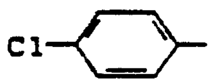 | $OCH_2$ | $nC_3H_7$ | Öl |
| 86 | I-86 | 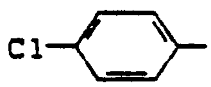 | $OCH_2$ | $iC_3H_7$ | Öl |
| 87 | I-87 | 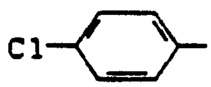 | $OCH_2$ | $nC_4H_9$ | Öl |
| 88 | I-88 | 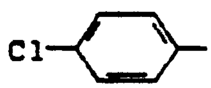 | $OCH_2$ | $nC_6H_{13}$ | Öl |
| 89 | I-89 | 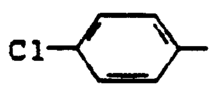 | $OCH_2-CH_2$ | $nC_3H_7$ | Öl |
| 90 | I-90 | 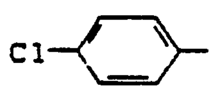 | $OCH_2-CH_2$ | $iC_3H_7$ | Öl |
| 91 | I-91 | 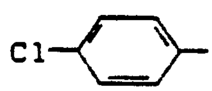 | $OCH_2-CH_2$ | $C_2H_5$ | Öl |

Tabelle 1 (Fortsetzung)

| Bsp. Nr. | Verb. Nr. | Ar | X | R | Schmelzpunkt [°C] bzw. Brechungsindex $[n_D^{20}]$ |
|---|---|---|---|---|---|
| 92 | I-92 | 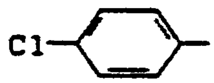 | $OCH_2$-$CH_2$ | $-CH_2-CH=CH_2$ | Öl |
| 93 | I-93 | 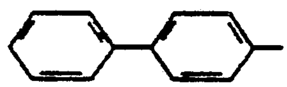 | O | 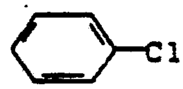 | Harz |
| 94 | I-94 | 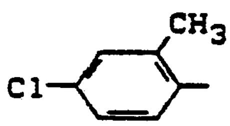 | O | 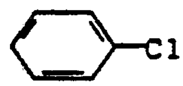 | Harz |
| 95 | I-95 | 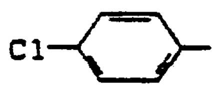 | O | $C_2H_5$ | 65 |
| 96 | I-96 | 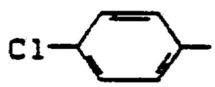 | O | 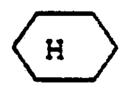 | 1.5405 |
| 97 | I-97 | 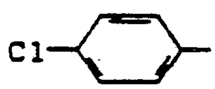 | O | n-$C_3H_7$ | 1.5398 |
| 98 | I-98 | 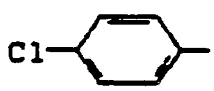 | O | i-$C_3H_7$ | 1.5320 |
| 99 | I-99 | 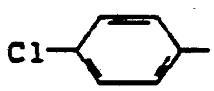 | O | n-$C_6H_{13}$ | Öl |
| 123 | I-100 | 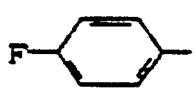 | $-CH_2-$ | $-CH_2-CH=CH_2-$ | 74-76 |
| 124 | I-101 | 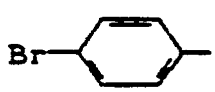 | $-OCH_2-$ | $-CH_2-CH=CH_2-$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 125 | I-102 | 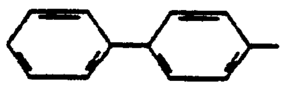 | $-O-CH_2-$ | $-CH_2CH=CH_2-$ | Öl |
| 126 | I-103 | 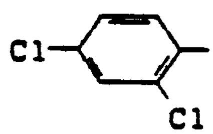 | $-O-CH_2-$ | $-CH_2CH=CH_2-$ | Öl |
| 127 | I-104 | 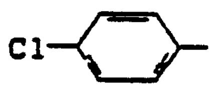 | $-O-CH_2-$ | $-CH_2CH=CH_2-$ | Öl |
| 128 | I-105 | 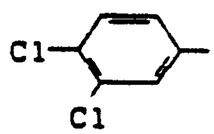 | $-O-CH_2-$ | $-CH_2CH=CH_2-$ | Öl |
| 129 | I-106 | 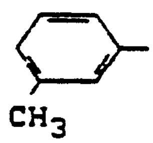 | $-CH_2-$ | $-CH_2CH=CH_2-$ | 66-68 |
| 130 | I-107 | 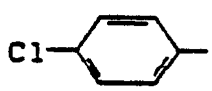 | -O- | $n-C_4H_9$ | 1.5349 |
| 131 | I-108 | 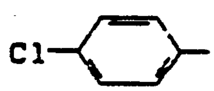 | -O- | $i-C_4H_9$ | 1.5310 |

Nach der im Beispiel 1 angegebenen Methode werden auch die in der folgenden Tabelle 2 aufgeführten Oxirane der Formel

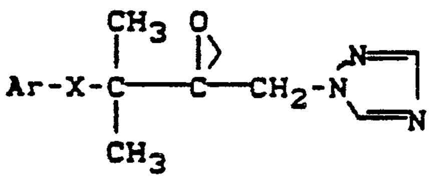

(II)

hergestellt.

Tabelle 2

| Bsp. Nr. | Verb. Nr. | Ar | X | Schmelzpunkt [°C] bzw. Brechungsindex $[n_D^{20}]$ |
|---|---|---|---|---|
| 100 | II-2 | 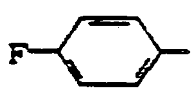 | $-CH_2-$ | Öl |
| 101 | II-3 | 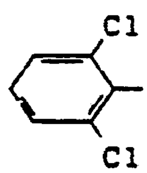 | $-OCH_2-$ | Öl |
| 102 | II-4 | 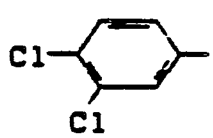 | $-OCH_2-$ | Öl |
| 103 | II-5 | 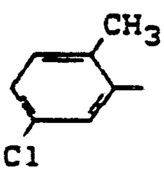 | $-OCH_2-$ | Öl |
| 104 | II-6 | 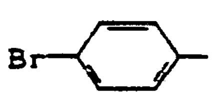 | $-OCH_2-$ | Öl |

Tabelle 2

| Bsp. Nr. | Verb. Nr. | Ar | X | Schmelzpunkt [°C] bzw. Brechungsindex [$n_D^{20}$] |
|---|---|---|---|---|
| 105 | II-7 | 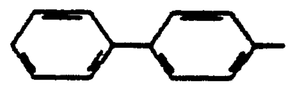 | $-OCH_2-$ | Öl |
| 106 | II-8 | 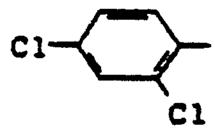 | $-OCH_2-$ | Öl |
| 107 | II-9 | 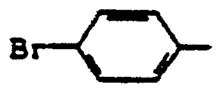 | $-CH_2-$ | Öl |
| 108 | II-10 | 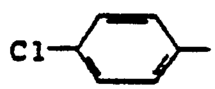 | $-CH_2-$ | Öl |
| 109 | II-11 | 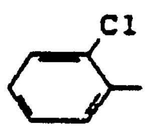 | $-CH_2-$ | Öl |
| 110 | II-12 | 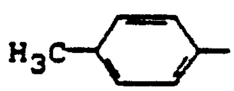 | $-CH_2-$ | Öl |
| 111 | II-13 | 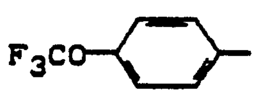 | $-CH_2-$ | Öl |
| 112 | II-14 | 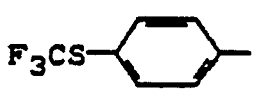 | $-CH_2-$ | Öl |
| 113 | II-15 | 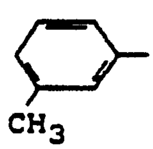 | $-CH_2-$ | Öl |
| 114 | II-16 | 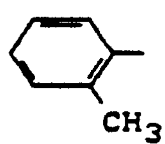 | $-CH_2-$ | Öl |

Tabelle 2 (Fortsetzung)

| Bsp. Nr. | Verb. Nr. | Ar | X | Schmelzpunkt [°C] bzw. Brechungsindex $[n_D^{20}]$ |
|---|---|---|---|---|
| 115 | II-17 | 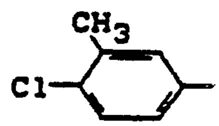 | $-OCH_2-$ | Öl |
| 116 | II-18 | 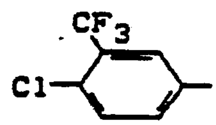 | $-CH_2-$ | Öl |
| 117 | II-19 | 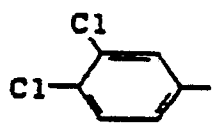 | $-CH_2-$ | Öl |
| 118 | II-20 | 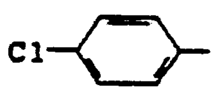 | $-OCH_2CH_2-$ | Öl |
| 119 | II-21 | 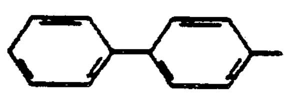 | -O- | Öl |
| 120 | II-22 | 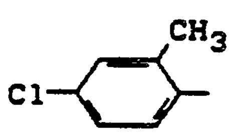 | -O- | Öl |
| 121 | II-23 | 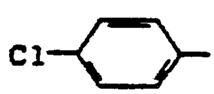 | -O- | Öl |

Verwendungsbeispiele:

In den nachfolgenden Verwendungsbeispielen werden die nachstehend angegebenen Substanzen als Vergleichsverbindungen eingesetzt (bekannt aus DE-OS 32 02 613).

(A) 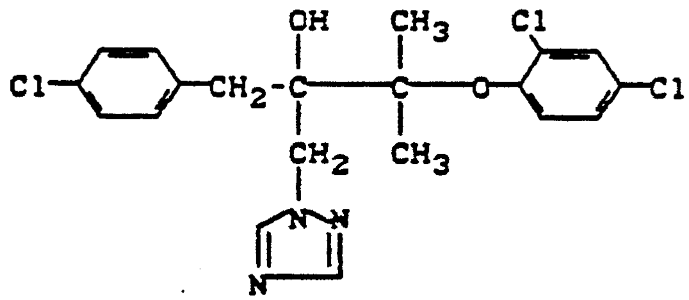

(B) 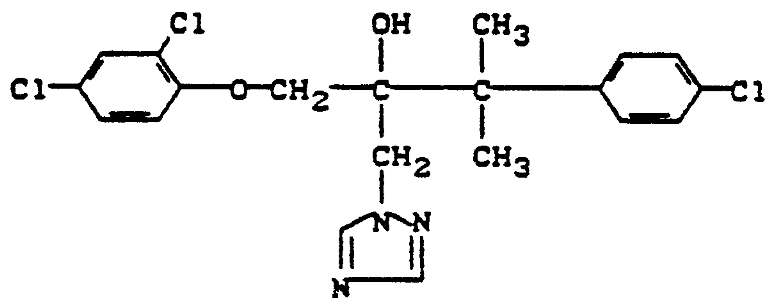

Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nähmedium dienten

a) für Dermatophyten und Schimmelpilze:

Sabouraud's milieu d'epreuve

b) für Hefen:

Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test zeigen die erfindungsgemäßen Verbindungen (I-2), (I-40), I-(41), (I-42), (1-43), (I-44), (I-45), (I-46), (I-47), (I-49), (I-50), (I-51), (I-52), (I-53)), (I-55), (I-58), (I-60), (I-61), (I-62), (I-63), (I-64), (I-65), (I-66), (I-67), (I-68), (I-69) und (I-70) eine bessere Wirksamkeit, insbesondere gegenüber Candida albicans, als die aus dem Stand der Technik bekannten Verbindungen (A) und (B).

Tabelle: A

Antimykotische in-vitro Wirksamkeit

| Wirk-Stoff | MHK-Werte in γ/ml Nährmedium | | | | |
|---|---|---|---|---|---|
| | Trichophyton mentagr. | Microsporum canis | Candida albicans | Torulopsis glabrata | Aspergillus fumigatus |
| (A) (bekannt) | <1 | 64 | 16 | 8 | 4 |
| (B) (bekannt) | <1 | 32 | 64 | 8 | >64 |
| Verbindungen gemäß Herstellungsbeispiel: | | | | | |
| I- 2 | <1 | 2 | <1 | 4 | 2 |
| I-40 | <1 | 2 | <1 | <1 | <1 |
| I-41 | <1 | <1 | <1 | 4 | <1 |
| I-42 | <1 | <1 | <1 | 8 | <1 |
| I-43 | <1 | <1 | <1 | 4 | <1 |
| I-44 | <1 | <1 | <1 | 4 | <1 |
| I-45 | <1 | <1 | 2 | 2 | <1 |
| I-46 | <1 | 4 | <1 | <1 | <1 |
| I-47 | <1 | 4 | 2 | 4 | <1 |
| I-49 | <1 | 2 | <1 | 2 | <1 |
| I-50 | <1 | <1 | <1 | 4 | <1 |
| I-51 | <1 | <1 | <1 | <1 | <1 |
| I-52 | <1 | 2 | <1 | 16 | <1 |
| I-53 | <1 | 2 | <1 | 2 | <1 |
| I-55 | <1 | 2 | 2 | 2 | 2 |
| I-58 | <1 | 4 | 2 | <1 | 8 |
| I-60 | <1 | 2 | 2 | 4 | <1 |
| I-61 | <1 | 2 | 8 | 32 | 2 |

Tabelle: A (Fortsetzung)

Antimykotische in-vitro Wirksamkeit

| Wirk-Stoff | MHK-Werte in γ/ml Nährmedium Tricho-phyton mentagr. | Micro-sporum canis | Candida albi-cans | Toru-lopsis glabrata | Asper-gillus fumigatus |
|---|---|---|---|---|---|
| I-62 | <1 | <1 | <1 | <1 | 2 |
| I-63 | <1 | 2 | 2 | 16 | <1 |
| I-64 | 2 | 2 | <1 | 4 | 4 |
| I-65 | 2 | 4 | 2 | 16 | 4 |
| I-66 | 4 | 4 | <1 | 8 | 4 |
| I-67 | <1 | 2 | <1 | 2 | 4 |
| I-68 | <1 | <1 | <1 | 4 | <1 |
| I-69 | <1 | <1 | <1 | 16 | 2 |
| I-70 | <1 | 2 | <1 | 8 | 8 |

Beispiel B

Antimykotische in-vivo-Wirksamkeit (oral) bei Mäusecandidose

Versuchsbeschreibung:

Mäuse vom Typ SPF-$CF_1$ wurden intravenös mit 1 - 2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 25 - 100 mg/kg Körpergewicht der Präparate oral behandelt.

Ergebnis:

Unbehandelte Tiere starben 3 bis 6 Tage post infektionem. Die Überlebensrate am 6.Tag post infektionem betrug bei unbehandelten Kontrolltieren etwa 5 %.

In diesem Test zeigen z.B. die erfindungsgemäßen Verbindungen (I-40), (I-46), (I-51), (I-52), (I-62), (I-93) und (I-94) eine bessere Wirkung als die aus dem Stand der Technik bekannten Verbindungen (A) und (B).

Zeichenerklärung:

| | | |
|---|---|---|
| +++++ | = sehr gute Wirkung | = 90 % Überlebende am 6.Tag.p.i. |
| ++++ | = gute Wirkung | = 80 % Überlebende am 6.Tag.p.i. |
| +++ | = Wirkung | = 60 % Überlebende am 6.Tag.p.i. |
| ++ | = schwache Wirkung | = 40 % Überlebende am 6.Tag.p.i. |
| + | = Spur Wirkung | = unter 40 % Überlebende am 6. Tag p.i. |
| k.W. | | = kein Unterschied zur unbehandelten Infektionskontrolle |

**Tabelle B**

**Antimykotische in-vivo-Wirksamkeit (oral) bei Mäuse-candidose**

| Wirkstoff | Wirkung |
|---|---|
| (A) (bekannt) | k.W. |
| (B) (bekannt) | k.W. |
| **Verbindungen gemäß Herstellungsbeispiel:** | |
| I-40 | +++ |
| I-46 | ++ |
| I-51 | ++ |
| I-52 | ++++ |
| I-62 | ++ |
| I-93 | +++++ |
| I-94 | ++++ |

Beispiel C

Antimikrobielle in-vivo-Wirksamkeit (lokal) am Modell der experimentellen Meerschweinchen-Trichophytie

Versuchsbeschreibung:

Weiße Meerschweinchen der Rasse Pirbright-white wurden auf dem geschorenen, nicht skarifizierten Rücken mit einer Mikro-und Makrokoniden-Suspension von Trichophyton mentagrophytes infiziert.

Die infizierten Tiere wurden beginnend mit dem 3.Tag p.i. 1 x täglich mit einer 0,1 %igen Lösung der erdingungsgemäßen Präparate (in Dimethylsulfoxid : Glycerin = 1 : 4) lokal behandelt.

Ergebnis:

Bei unbehandelten Tieren entwickelte sich innerhalb von 12 Tagen p.i. das Typische Bild einer Dermatophytose mit Rötung, Schuppung und Haarausfall bis zum totalen Integument-Defekt an der Infektionsstelle.

In diesem Test zeigen z.B. die erfindungsgemäßen Verbindungen (I-40) gute und (I-52) sehr gute Wirkung.

**Tabelle C**

**Antimykotische in-vivo-Wirksamkeit (lokal) am Modell der experimentellen Meerschweinchen-Trichophytie**

| Wirkstoff | Wirkung |
|---|---|
| Verbindung gemäß Herstellungsbeispiel: | |
| (I-40) | ++++ |
| (I-52) | +++++ |

Erläuterung:

+++++ = sehr gute Wirkung = keine Infektionszeichen am 12. bis 15. Tag p.i.

++++ = gute Wirkung = geringe Rötung, vereinzelt Schuppen

+++ = Wirkung = Rötung, Schuppen ohne Haarausfall

++ = schwache Wirkung = Rötung, Schuppen, Haarausfall

+ = Spur Wirkung = flächiger Haarausfall, entzündliche Hautreaktion

## Beispiel D / Formulierungen

### 1.) Lösung:

| | | |
|---|---|---|
| Wirkstoff gemäß Formel (I) | : | 10 g |
| Alkohol, rein (96 %ig) | : | 300 g |
| Isopropylmyristat | : | 526 g |
| | | 836 g |

### 2.) Creme:

| | | |
|---|---|---|
| Wirkstoff gemäß Formel (I) | : | 10 g |
| Aralcel 60 (Sorbitan-monostearat) | : | 20 g |
| Tween 60 (Polyoxyethylen (2) -sorbitan-monostearat) | : | 15 g |
| Walrat, künstlich (Mischung vom Estern von gesättigten Fettsäuren $C_{14}$-$C_{18}$ und Fettalkoholen $C_{14}$-$C_{18}$ | : | 30 g |
| Lanette O | : | 100 g |
| Eutanol G (2-Octyl-dodecanol) | : | 135 g |
| Benzylalkohol | : | 10 g |
| Wasser, entmineralisiert | : | 680 g |
| | | 1000 g |

**Ansprüche**

1. Mercapto-substituierte Hydroxyethyl-(triazol-1-yl)-Derivate der Formel

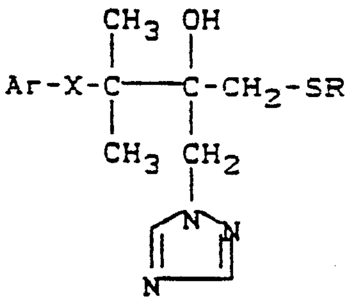

(I)

in welcher
Ar für gegebenenfalls substituiertes Aryl steht,
X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2-$steht, und
R für Wasserstoff, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl oder für gegebenenfalls substituiertes Aralkyl oder gegebenenfalls substituiertes Phenyl steht,
sowie deren Säureadditions-Salze und Metallsalz-Komplexe zur Behandlung von Krankheiten.

2. Mercapto-substituierte Hydroxyethyl-(triazol-1-yl)-Derivate der Formel I in Anspruch 1, in denen
Ar für Aryl mit 6 bis 10 Kohlenstoffatomen steht, welches einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone,
X für Sauerstoff, Schwefel, eine direkte Bindung sowie für die Gruppierungen$-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-$oder $-S-CH_2-CH_2-$steht, und
R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlenstoffatomen, gegebenenfalls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone oder gegebenenfalls durch Halogen und/oder Akyl substituiertes Phenyl steht, zur Behandlung von Krankheiten.

3. Mercapto-substituierte Hydroxyethyl-(triazol-1-yl)-Derivate der Formel I in Anspruch 1, in denen
Ar für Phenyl steht, welches einfach bis dreifach, gleichartig oder verschieden substituiert sein kann durch Halogen, Alkyl mit 1 bis 4 Kohlenstoffatomen, Halogenalkyl mit 1 oder 2 Kohlen stoffatomen und 1 bis 5 Halogenatomen, Halogenalkoxy mit 1 oder 2 Kohlenstoffatomen und 1 bis 5 Halogenatomen und/ oder Halogenalkylthio mit 1 oder 2 Kohlenstoffatomen und 1 bis 5 Halogenatomen und/oder Phenyl
X für Sauerstoff, Schwefel, eine direkte Bindung sowie für die Gruppierungen $-CH_2-$oder $-CH_2-CH_2-$steht, und
R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlenstoffatomen, gegebenenfalls

substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone oder für ein-bis dreifach durch Halogen und/oder $C_1$-$C_4$-Alkyl substituiertes Phenyl steht, zur Behandlung von Krankheiten.

4. Mercapto-substituierte Hydroxyethyl-(triazol-1-yl)-Derivate der Formel I in Anspruch 1, in denen

Ar für Phenyl steht, welches einfach bis dreifach, gleichartig oder verschieden substituiert sein kann durch Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl Formyl und Acetyl und/oder von Formyl oder Acetyl abgeleitete Oximether,

X für die Gruppierungen $-O-CH_2-$, $-S-CH_2-$, $-O-CH_2-CH_2-$oder $-S-CH_2-CH_2-$steht, wobei das Heteroatom jeweils mit dem Aryl-Rest verbunden ist, und

R für Wasserstoff, gegebenenfalls substituiertes, geradkettiges oder verzweigtes Alkyl mit 1 bis 12 Kohlenstoffatomen, gegebenenfalls substi tuiertes Alkenyl mit 2 bis 12 Kohlenstoffatomen, gegebenenfalls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone oder für gegebenenfalls ein-bis dreifach durch Halogen und/oder $C_1$-$C_4$-Alkyl substituiertes Phenyl steht, zur Behandlung von Krankheiten.

5. Mercapto-substituierte Hydroxyethyl-(triazol-1-yl)-Derivate der Formel I in Anspruch 1, in denen

Ar für phenyl steht, welches einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto und/oder Phenyl,

X für Sauerstoff sowie für die $-CH_2$-Gruppe steht und

R für Wasserstoff, gegebenenfalls durch Halogen substituiertes Alkyl mit 1 bis 6 Kohlenstoffatomen, gegebenenfalls durch Halogen sustituiertes Alkenyl mit 2 bis 4 Kohlenstoffatomen, gegebenenfalls durch Halogen substituiertes Alkinyl mit 3 bis 6 Kohlenstoffatomen, gegebenenfalls ein-oder zweifach, gleichartig oder verschieden durch Fluor, Chlor und/oder Methyl substituiertes Cycloalkyl mit 3 bis 6 Kohlenstoffatomen, gegebenenfalls ein-oder zweifach, gleichartig oder verschieden durch Fluor, Chlor und/oder Methyl substituiertes Cycloalkylalkyl mit 3 bis 6 Kohlenstoffatomen im Cycloalkylteil und 1 oder 2 Kohlenstoffatomen im Alkylteil oder für Benzyl, welches im Phenylteil einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluor methylmercapto und/oder Tetrafluorethylmercapto steht oder für gegebenenfalls ein-oder zweifach durch Fluor, Chlor und/oder Methyl substituiertes Phenyl steht, zur Behandlung von Krankheiten.

6. Mercapto-substituierte Hydroxyethyl-(triazol-1-yl)-Derivate der Formel I in Anspruch 1, in denen

Ar für Phenyl steht, welches einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, Formyl, Acetyl, Methoximinomethyl, Ethoximinomethyl, Methoximinoethyl, Ethoximinoethyl und/oder Phenyl,

X für die Gruppierungen $-O-CH_2-$, $-S-CH_2-$, $-O-CH_2-CH_2-$oder $-S-CH_2-CH_2-$steht, wobei das Heteroatom mit dem Arylrest verbunden ist, und

R für Wasserstoff, gegebenenfalls durch Halogen substituiertes Alkyl mit 1 bis 6 Kohlenstoffatomen,

gegebenenfalls durch Halogen subtituiertes Alkenyl mit 2 bis 4 Kohlenstoffatomen, gegebenenfalls durch Halogen substituiertes Alkinyl mit 3 bis 6 Kohlenstoffatomen, gegebenenfalls ein-oder zweifach, gleichartig oder verschieden durch Fluor, Chlor und/oder Methyl substituiertes Cycloalkyl mit 3 bis 6 Kohlenstoffatomen, gegebenenfalls ein-oder zweifach, gleichartig oder verschieden durch Fluor, Chlor und/oder Methyl substituiertes Cycloalkylalkyl mit 3 bis 6 Kohlenstoffatomen im Cycloalkylteil und 1 oder 2 Kohlenstoffatomen im Alkylteil oder für Benzyl, welches im Phenylteil einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto und/oder Tetrafluorethylmercapto oder für gegebenenfalls ein-oder zweifach durch Fluor, Chlor und/oder Methyl substituiertes Phenyl steht, zur Behandlung von Krankheiten.

7. Mercapto-substituierte Hydroxyethyl-(triazol-1-yl)-Derivate der Formel I in Anspruch 1, in denen

Ar für Phenyl steht, welches einfach oder zweifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, Formyl, Acetyl, Methoximinomethyl, Ethoximinomethyl, Methoximinoethyl, Ethoximinoethyl und/oder Phenyl,

X für die Gruppierungen $-O-CH_2-$und $-O-CH_2-CH_2-$steht, wobei das Heteroatom mit dem Arylrest verbunden ist, und

R für Wasserstoff, Methyl, Ethyl, i-Propyl, n-Propyl, i-Butyl, n-Butyl, t-Butyl, n-Hexyl, Cyclohexyl, Allyl, Propinyl, Dichlorcyclopropyl, Dichlorcyclopropyl-methyl, Methylcyclohexyl, 4-Chlorbenzyl oder 4-Chlorphenyl steht zur Behandlung von Krankheiten.

8. Verbindungen der Formel I in Anspruch 1 worin

Ar vorzugsweise für Phenyl steht, welches einfach oder zweifach, gleichartig oder verscheiden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, und/oder Phenyl,

X für Sauerstoff sowie für die Gruppierung $-CH_2-$steht und

R für Wasserstoff, Methyl, Ethyl, i-Propyl, n-Propyl, i-Butyl, t-Butyl n-Hexyl, Cyclohexyl, Allyl, Propinyl, Dichlorcyclopropylmethyl, Methylcyclohexyl, 4-Chlorbenzyl oder 4-Chlorphenyl steht zur Behandlung von Krankheiten.

9. Verwendung von mercapto-substituierten Hydroxyethyl-(triazol-1-yl)-Derivaten der Formel I mit der in einem oder mehreren der Ansprüche 1 bis 8 angegebenen Bedeutung zur Herstellung von Arzneimitteln zur Bekämpfung von Mykosen.